# EUROPEAN PATENT APPLICATION

(11) **EP 4 410 283 A1**
(43) Date of publication of application: **07.08.2024**
(21) Application number: 23382078.6
(22) Date of filing: 31.01.2023
(51) Int. Cl.: A61K 31/138, A61K 31/517, A61K 31/713, A61K 45/06, A61K 47/55, A61P 35/00

(54) **TRIPLE COMBINED THERAPY INHIBITING EGFR, RAF1 AND STAT3 AGAINST PANCREATIC DUCTAL ADENOCARCINOMA**

(71) Applicant: Fundación del Sector Público Estatal Centro Nacional de Investigaciones Oncológicas Carlos III (F.S.P. CNIO), 28029 Madrid (ES)
(72) Inventor: BARBACID, Mariano, 28029 Madrid (ES); GUERRA, Carmen, 28029 Madrid (ES); LIAKI, Vasiliki, 28029 Madrid (ES)
(74) Representative: Clarke, Modet y Cía., S.L.

(57) **Abstract**

The present invention relates to a pharmaceutical composition comprising an inhibitor of the expression, activity and/or function of c-Raf, an inhibitor of the expression, activity and/or function of EGF Receptor (EGFR), and an inhibitor of the expression, activity and/or function of STATS and its use in the treatment of pancreatic cancer.

## Description

### FIELD OF THE INVENTION

The present invention relates to a combined therapy against cancer which has proven to be particularly useful in the prevention and treatment of pancreatic cancer.

### BACKGROUND

Pancreatic cancer is one of the most lethal cancer types, as it accounts for almost as many deaths as cases, and it has been projected as the second highest in mortality by 2030. With a 5-year survival rate of only 5%, the median survival is less than 6 months. However, minimal improvements have been made in the field of treatment. Currently, surgical resection remains the only curative option for early-stage local disease, which corresponds to 20-30% of patients. Unfortunately, due to late diagnosis, most patients cannot benefit from surgery, as at the time of diagnosis, they display advanced disease with metastasis. Cytotoxic chemotherapy is the standard treatment, with overall survival in the range of weeks to few months (Nevala-Plagemann *et al.,* 2020). At present, no targeted therapeutic approaches are available for pancreatic ductal adenocarcinoma (PDAC) patients. So far, adjuvant treatment with 5-fluorouracil, leucovorin, irinotecan and oxaliplatin (FOLFIRINOX) has been demonstrated to provide the longest median overall survival (OS) (54 months) in patients with resectable disease (Conroy *et al.,* 2018). For patients with advanced-stage or metastatic disease, the standard of care consists of modern combinations of highly cytotoxic agents such as nab-paclitaxel plus gemcitabine or FOLFIRINOX, but only provides modest improvements in OS in the range of weeks to months (Conroy *et al.,* 2018; Von Hoff *et al.,* 2013). Undoubtedly, there is urgent need to develop novel targeted therapies that directly block specific oncogenic pathways with reduced toxicity. Indeed, the recent consensus statement from the United States National Cancer Institute (NCI) indicated the need for targeted agents, predictive biomarkers, and improved preclinical models for PDAC (Philip *et al.,* 2009).

Few preclinical studies have published combinations of EGFR or STAT3 inhibition with other compounds. In 2011, Nagaraj et al. described that only the combination of dasatinib and erlotinib with gemcitabine overcame STAT3-mediated resistance of EGFR and Src inhibition both in vitro and in vivo. The pharmacological combination used in this study only includes the EGFR inhibitor Erlotinib (Nagaraj et al., 2011). In 2012, Navas et al. demonstrated that the inhibition of EGFR signaling with Erlotinib alone is not sufficient to target pancreatic cancer. Also, combination of phosphatidylinositol 3-kinase (PI3K) inhibitor with Stat3 shRNA (short hairpin RNA or small hairpin RNA (ribonucleic acid)) robustly inhibited proliferation of all tumor cell explants (Navas et al., 2012). In 2015, Zhou et al. demonstrated that STAT3 was significantly activated following MEK inhibition using AZD6244, PD98059 and Trametinib in Kras mutant pancreatic and colon cancer cells. As a result, dual inhibition of STAT3 and MEK exerts significant anti-tumor cell efficacy in Kras mutant pancreatic cancer cells *in vitro* (Zhao et al., 2015). In 2017, Sahu et al. disclosed that MAP2K (MEK) inhibitors were most effective in targeting PDAC spheroids, while combination with the multikinase inhibitor ponatinib was effective in targeting pancreatic cancer cells both in monolayer and spheroids. They also demonstrated that using xenograft models, combined treatment with a MEK inhibitor and ponatinib causes significant tumor regression. PDAC patient samples also provided evidence of increased STAT3 activation in PDAC tumors and MAPK1 (ERK) activation in liver metastases, implicating STAT3 and ERK as key drivers in primary tumors and metastases, respectively (Sahu et al., 2017). In 2019, Blasco et al. demonstrated that genetic ablation of *Egfr* and *Raf1* results in complete regression of a significant fraction of *Kras*/*Trp53* driven tumors of small sizes. Yet, some small tumors and most tumors of bigger sizes remained refractory to this combined deletion (Blasco *et al.,* 2019 and WO2020020942 A1).

### DESCRIPTION OF THE INVENTION

The inventors have shown that the simultaneous inhibition of the expression, activity and/or function of EGFR, RAF1 and STAT3 in PDAC results in a significant therapeutic effect with an extremely low toxicity. Previous studies with a double therapy against EGFR and RAF1 showed that this therapy was not effective in advanced and more aggressive PDACs of bigger sizes, which do not respond to the double combination. However, these advanced and more aggressive PDACs of bigger sizes can be effectively targeted with the triple combined therapy. The present invention represents an improved therapy in the treatment of pancreatic cancer since it leads to a complete tumor regression while its side effects are much smaller than other therapies described up to date.

In a first aspect, the present invention relates to a pharmaceutical composition comprising:
a. an inhibitor of the expression, activity and/or function of c-Raf;
b. an inhibitor of the expression, activity and/or function of EGF Receptor (EGFR);
c. an inhibitor of the expression, activity and/or function of STAT3.

In a preferred embodiment of the first aspect of the present invention, the inhibitor of the expression, activity and/or function of each one of c-Raf, EGFR or STAT3 comprises at least an inhibitor selected form: an inhibitor compound, an inhibitor antibody or an antigen binding fragment thereof, a peptide, a nucleotide sequence, a proteolysis targeting chimera (PROTAC), and any combination thereof.

In a preferred embodiment of the first aspect of the present invention, when the inhibitor of the expression, activity and/or function of c-Raf, EGFR or STAT3 comprises a nucleotide sequence, the nucleotide sequence is or codifies for a guide RNA, an interfering RNA, such as a short hairpin RNA or small hairpin RNA (shRNA) or a micro RNA.

In a preferred embodiment of the first aspect of the present invention, the inhibitor of the expression, activity and/or function of c-Raf does not inhibit the expression, activity and/or function of b-Raf. In a preferred embodiment of the first aspect of the present invention, the inhibitor of the expression, activity and/or function of c-Raf does not inhibit C-RAF kinase activity. In a preferred embodiment, the inhibitor of the expression, activity and/or function of c-Raf inhibits the expression of c-Raf or promotes C-RAF degradation. In another preferred embodiment, the inhibitor of the expression, activity and/or function of c-Raf blocks C-RAF kinase independent activities.

In a preferred embodiment, the present invention relates to a pharmaceutical composition comprising an inhibitor of the expression, activity and/or function of c-Raf and an inhibitor of the expression, activity and/or function of EGF Receptor (EGFR), wherein the inhibitor of the expression, activity and/or function of c-Raf:
(i) does not inhibit C-RAF kinase activity;
(ii) inhibits the expression of c-Raf;
(iii) promotes C-RAF degradation; and/or
(iv) blocks C-RAF kinase independent activities.

In a preferred embodiment of the first aspect of the present invention, the inhibitor of the expression, activity and/or function of c-Raf is other than sorafenib, vemurafenib, dabrafenib y LY3009120. In a preferred embodiment, the inhibitor of the expression, activity and/or function of c-Raf promotes C-RAF degradation. In a more preferred embodiment, said inhibitor is a proteolysis targeting chimera (PROTAC).

In a preferred embodiment of the first aspect of the present invention, the inhibitor of the expression, activity and/or function of EGFR is selected from afatinib, erlotinib, gefitinib, brigatinib, icotinib, neratinib, lapatinib, vandetanib, osimertinib, cetuximab, panitumumab, necitumumab, nimotuzumab, zalutumumab, matuzumab and combinations thereof.

In a preferred embodiment of the first aspect of the present invention, the inhibitor of the expression, activity and/or function of STAT3 is a shRNA or a PROTAC.

In a preferred embodiment of the first aspect of the present invention, the composition comprises a therapeutically effective amount of an inhibitor of the expression, activity and/or function of c-Raf, a therapeutically effective amount of an inhibitor of the expression, activity and/or function of EGFR, a therapeutically effective amount of an inhibitor of the expression, activity and/or function of STAT3 and optionally a pharmaceutically acceptable excipient.

In another aspect, the present invention relates to the pharmaceutical composition of the first aspect for use in the prevention and/or treatment of cancer. Preferably, for use in the prevention and/or treatment of pancreatic cancer. The present invention relates also to the pharmaceutical composition of the first aspect for use in the prevention and/or treatment of pancreatic intraepithelial neoplasia or of pancreatic ductal adenocarcinoma. Preferably, the present invention relates to the pharmaceutical composition of the first aspect for use in tumor regression in a subject afflicted with said cancer.

The present invention relates also to the pharmaceutical composition of the first aspect for use in tumor regression in a subject afflicted with pancreatic ductal adenocarcinoma (PDAC). The present invention relates also to the pharmaceutical composition of the first aspect for use in tumor regression in a subject who does not respond to a composition comprising only the combination of an inhibitor of the expression, activity and/or function of c-Raf and an inhibitor of the expression, activity and/or function of EGFR.

An aspect of the present invention is a method for the prevention and/or treatment of cancer comprising the administration of a therapeutically effective amount of an inhibitor of the expression, activity and/or function of c-Raf, a therapeutically effective amount of an inhibitor of the expression, activity and/or function of EGF Receptor (EGFR), and a therapeutically effective amount of an inhibitor of the expression, activity and/or function of STAT3 and optionally a pharmaceutically acceptable excipient. Preferably, the method of the present invention is for the treatment and/or prevention of pancreatic cancer, more preferably of pancreatic intraepithelial neoplasia (PanIN) or of PDAC.

### DESCRIPTION OF THE FIGURES

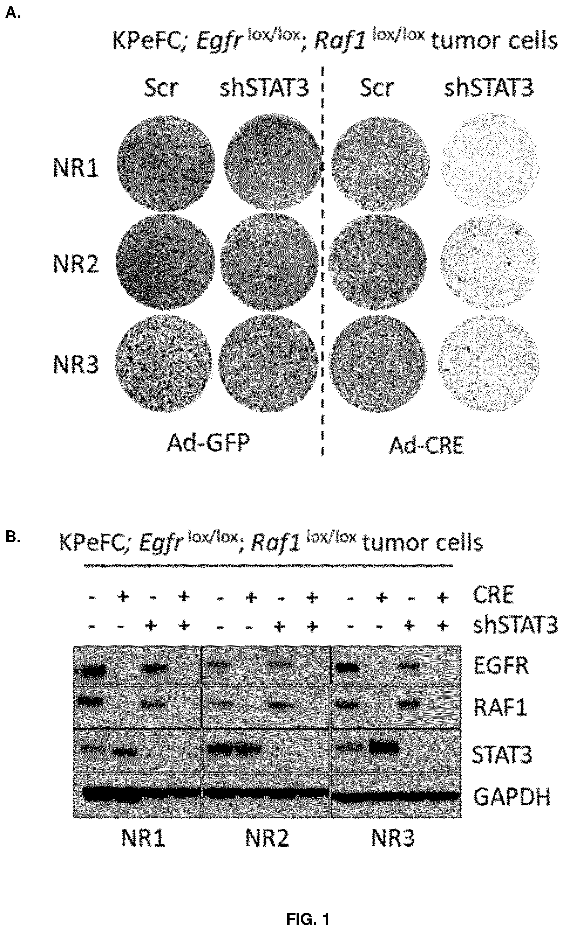

**Figure 1****.** Colony formation assay of Egfr^{lox/lox}; Raf1^{lox/lox} PDAC cells infected with Adeno-GFP and Adeno-CRE viral particles in combination with small-hairpin RNA (shRNA) against STAT3 and Scr (empty vector) as control. 5000 cells were seeded for 10 days. **B.** Western blot analysis of the expression of EGFR, RAF1 and STAT3 of cell-lysate extracts. GAPDH was used as loading control.

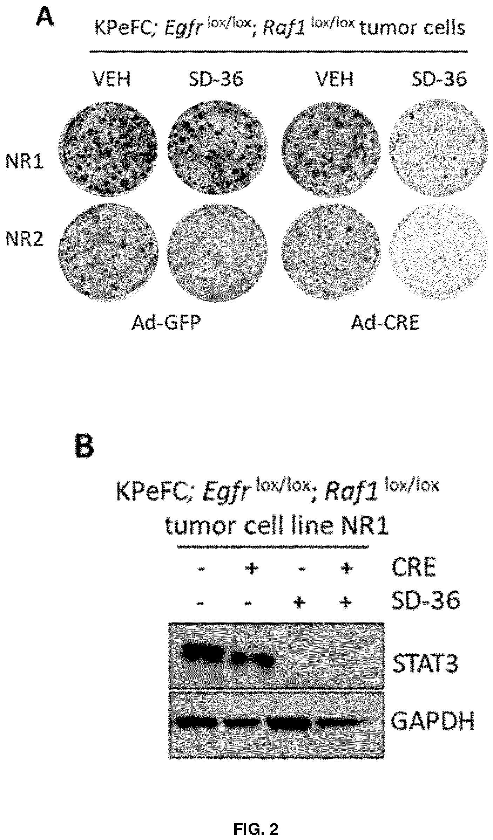

**Figure 2****. A.** Colony formation assay of Egfr^{lox/lox}; Raf1^{lox/lox} PDAC cells infected with Adeno-GFP and Adeno-CRE viral particles in combination with treatment with 1µM SD-36. 5000 cells were seeded for 10 days and culture medium was changed every day. **B.** Western blot analysis of the expression of STAT3 of cell-lysate extracts. GAPDH was used as loading control.

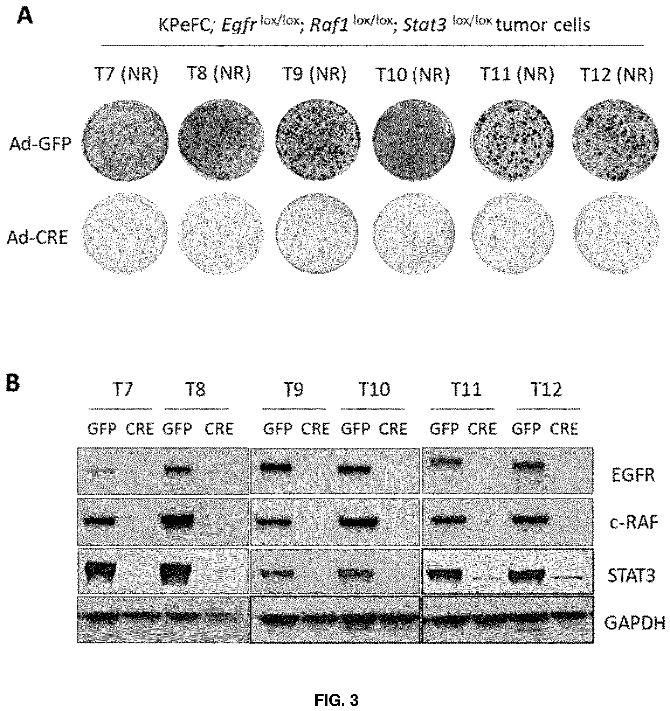

**Figure 3****.** Colony formation assay of Egfr^{lox/lox}; Raf1^{lox/lox}; Stat3^{lox/lox} PDAC cells infected with Adeno-GFP and Adeno-CRE viral particles. 5000 cells were seeded for 10 days. **B.** Western blot analysis of the expression of EGFR, c-RAF and STAT3 of cell-lysate extracts. GAPDH was used as loading control.

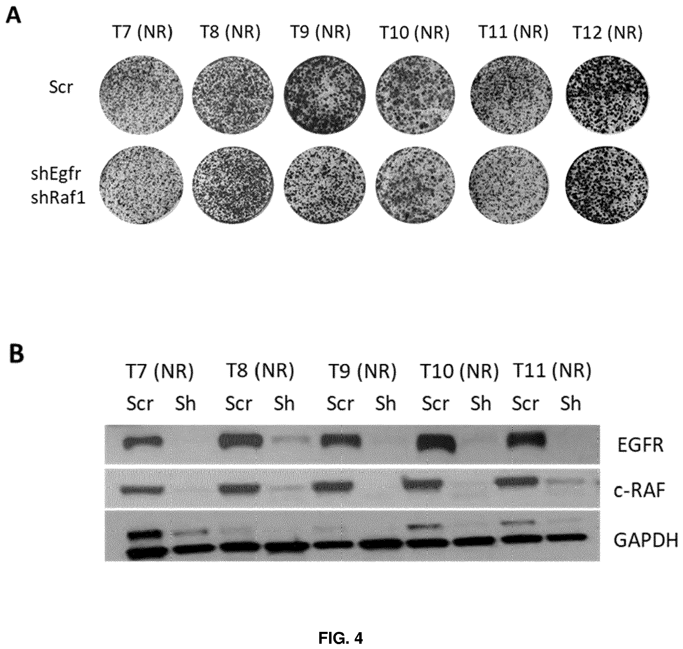

**Figure 4****. A.** Colony formation assay of Egfr^{lox/lox}; Raf1^{lox/lox}; Stat3^{lox/lox} PDAC cells (T7-T12 cell lines) infected with shRNA against EGFR and RAF1 and Scr (empty vector) as control. 5000 cells were seeded for 10 days. **B.** Western blot analysis of the expression of EGFR and RAF1 of cell-lysate extracts. GAPDH was used as loading control.

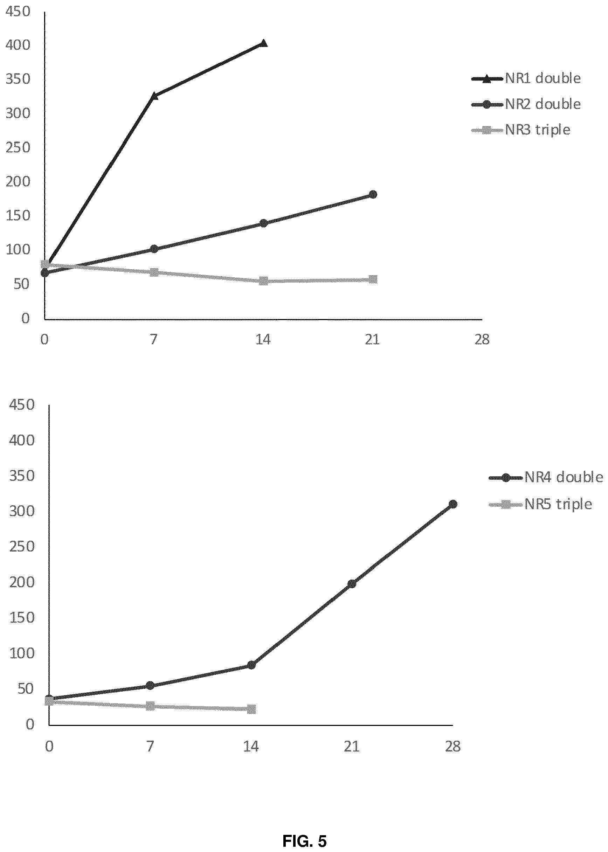

**Figure 5****.** Tumor growth monitored by ultrasound of genetically engineered mice with genotype (*Elas-tTa*/*tetO-Flp; K*-*Ras*^{+/FSFG12V}; *p53*^{frt/frt}; *Raf1* ^{lox/lox}; *Rosa26_CreERT2* ^{+/KI}) treated with tamoxifen and afatinib (NR double) and tamoxifen, afatinib and SD-36 (NR triple).

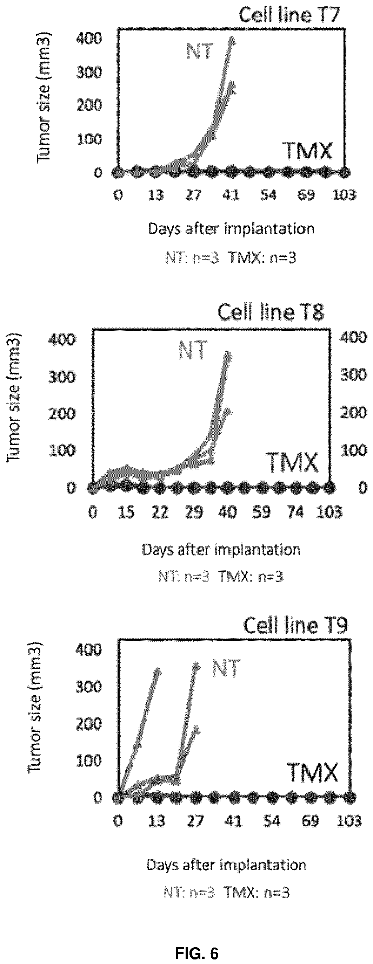

**Figure 6****.** Tumor growth monitored by ultrasound after implantation of three Egfr^{lox/lox}; Raf1^{lox/lox}; Stat3^{lox/lox} PDAC cell lines (T7-T9) in triplicates. NT: non-treated mice before and after implantation of cells. TMX: tamoxifen-treated mice, subjected to tamoxifen diet one week before the implantation of cells until the end of the experiment. NT mice were sacrificed at a humane end point due to tumor burden at the last timepoint indicated in the graph.

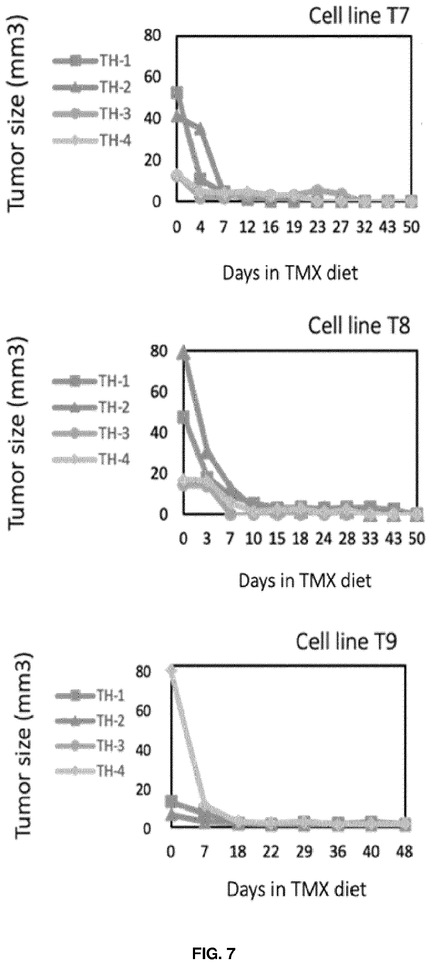

**Figure 7****.** Tumor growth monitored by ultrasound of mice implanted with three Egfr^{lox/lox}; Raf1^{lox/lox}; Stat3^{lox/lox} PDAC cell lines (T7-T9) during the time that were under tamoxifen diet.

### EXAMPLES

The present invention is exemplified and illustrated by the following examples, which are not intended to limit in any way the present invention:

### Materials and methods

Silencing of *Egfr, Raf1* and *Stat3* was mediated with the lentiviral short hairpin RNA (shRNA) plasmids TRCN0000055218, TRCN0000012628 and TRCN0000071453 respectively. The non-target shRNA vector was used as control. The pharmacological treatments were conducted with the following agents: Afatinib (20 mg/kg, daily), SD-36 (50 mg/kg, daily).

### The triple elimination of EGFR, RAF1 and Stat3 eliminated all PDACs

Combined ablation of EGFR and RAF1 expression resulted in complete regression of a significant percentage (around 50%) of PDAC tumors (Responder, R, tumors) driven by Kras/Trp53 mutations in genetically engineered mice (Blasco et al., Cancer Cell 2019). The experimets were done in tumors where the oncogene Kras is mutated as well as the oncosupressor gene p53 (For short KPeFC in figures). Basically, the cases of pancreatic ductal adenocarcinoma, both in human and in mouse models, have two major drivers: Oncogenic Kras mutations and p53 mutations. In the study of Blasco et al., 50% of the mouse PDACs (Non responder, NR, tumors) were insensitive to this therapeutic strategy. Surprisingly, the triple elimination of EGFR, RAF1 and Stat3 eliminated all PDACs: R and NR tumors.

### STAT3 inhibition (genetic or pharmacological) resulted in cell death of NR tumor cells

The expression of STAT3 was targeted by shRNAs after genetic deletion of *Egfr* and *Raf1* in NR cells by Adeno-GFP and Adeno-GFP-CRE infection. Importantly, this combined therapeutic strategy resulted in cell death of NR tumor cells. Notably, downregulation of Stat3 did not affect NR cells that express *Egfr* and *Raf1* (Figure 1 A, B). These results were further validated by a pharmacological approach, using the STAT3 PROTAC degrader SD-36 (Figure 2 A, B). NR tumor cells, previously infected with Adeno-GFP and Adeno-GFP-CRE virus to eliminate the EGFR and the Raf1 (floxed targets), were treated with SD-36 for 10 days in a colony formation assay. Elimination of the targets was very efficient. SD-36 effectively degraded STAT3 and has no toxic side-effects, as NR cells that express Egfr and Raf1 grew similarly as the vehicle control.

### Triple deletion in vitro in Non Responder cells

We performed also a genetic approach: triple elimination of *Egfr, Raf1* and *Stat3.* For this, we generated a PDAC mouse model that carries the tree floxed alleles: Egfr^{lox/lox}; Raf1^{lox/lox}; Stat3^{lox/lox}. 25 PDAC cell lines were generated for in vitro studies. The simultaneous elimination of the three floxed targets by Adeno-CRE infection resulted in complete cell death of the 25 cell lines. Figure 3 A, B shows the example of 6 cell lines. Interestingly, the few small colonies that remained were mostly apoptotic and could not be collected for further studies.We identified these cell lines as NR based on their answer to the elimination of EGFR and Raf1 by shRNAs (Figure 4 A, B). From the 25 cell lines, 12 were NR. Therefore, Elimination of *the three targets efficiently* induced cell death in NR cells.

### Triple deletion in vivo

We also studied the potential therapeutic value of the simultaneous elimination of *Egfr, Raf1* and *Stat3 in vivo.* We performed orthotopic studies, by inoculating three KPeFC; Egfr^{lox/lox}; Raf1^{lox/lox}; Stat3^{lox/lox} tumor cell lines in the pancreas of immunocompetent mice (C57BU6) mice. These cell lines were NR to the elimination of *Egfr* and *Raf1* (T7-T9, Figure 4 A, B) and carry the Rosa26CreERT2 allele. Upon exposure to tamoxifen, CreERT2 recombinase was activated resulting in the elimination of the floxed alleles in the tumor cells. We performed two different protocols for tamoxifen treatment (supplied in the diet) to study the effect of the triple elimination: right after implantation and in established tumors. For the first protocol, tamoxifen treatment started one week before the day of the engraftment of cells in the pancreatic parenchyma. This approach achieved very early deletion of the targets, before tumor detection (orthotopic initiation study). Monitoring of tumor growth by ultrasound showed that tumors developed in non-treated mice in less than 30 days, while in mice under tamoxifen diet there was no tumor growth during the time of the study (100 days) (Figure 5). Therefore, elimination of the three targets *in vivo* resulted in tumor cell death. For a more therapeutic approach, we performed an orthotopic therapeutic study, in which the tamoxifen diet was provided to tumor bearing mice. Strikingly, we observed regression of tumors of different sizes (10-80 mm³) after a treatment period of approximately two weeks. Tumor monitoring was continued for additional 35 days under tamoxifen treatment to confirm that triple elimination in the tumoral cells induced complete and sustained tumor regression (Figure 6). Therefore, the simultaneous elimination of the three targets *in vivo* produced complete tumor regression.

Upon treatment with tamoxifen, the expression of the gene Raf1 was eliminated genetically. It was also included in the combined treatment Afatinib (a tyrosine receptor kinase inhibitor against EGFR) and SD-36 (PROTAC degrader against STAT3). Interestingly, double inhibition of EGFR and RAF1 with Afatinib and Tamoxifen didn't cause any delay in the tumor growth of tumors of big sizes (NR double). However, additional treatment with SD-36 (NR triple) led to significant delay and slight reduction of tumor growth, validating the therapeutic value of the triple inhibition in NR PDACs compared to the double combination.

### REFERENCES

Blasco, T.M. et al. (2019). Cancer Cell 35, 573-587.
Conroy, T. et al. (2018). N. Engl. J. Med. 379, 2395-2406.
Navas, C. et al. (2012). Cancer Cell. 22, 318-30.
Nagaraj, N.S. et al. (2011). Clin Cancer Res. 17, 483-93.
Nevala-Plagemann, C. et al. (2020). Nat. Rev. Clin. Oncol. 2, 108-123.
Philip, P.A. et al. (2009). J Clin Oncol. 27, 5660-9.
Sahu, N. et al. (2017). Mol Cancer Ther. 9, 1729-1738.
Von Hoff, D.D. et al. (2013). N Engl J Med. 369,1691-703.
Zhao, C. et al. (2015). Oncotarget 6, 14472-87.

## Claims

1. A pharmaceutical composition comprising:
a. an inhibitor of the expression, activity and/or function of c-Raf;
b. an inhibitor of the expression, activity and/or function of EGF Receptor (EGFR);
c. an inhibitor of the expression, activity and/or function of STAT3.

2. The pharmaceutical composition according to claim 1, wherein the inhibitor of the expression, activity and/or function of each one of c-Raf, EGFR and STAT3 comprises at least an inhibitor selected from: an inhibitor compound, an inhibitor antibody or an antigen binding fragment thereof, a peptide, a nucleotide sequence, a proteolysis targeting chimera (PROTAC), and any combination thereof.

3. The pharmaceutical composition according to claim 2, wherein the nucleotide sequence is or codifies for a guide RNA, an interfering RNA, shRNA, or a micro RNA.

4. The pharmaceutical composition according to any one of claims 1 to 3, wherein the inhibitor of the expression, activity and/or function of c-Raf is other than sorafenib, vemurafenib, dabrafenib y LY3009120.

5. The pharmaceutical composition according to any one of claims 1 to 4, wherein the inhibitor of the expression, activity and/or function of c-Raf does not inhibit the expression, activity and/or function of b-Raf; and/or wherein the inhibitor of the expression, activity and/or function of c-Raf does not inhibit C-RAF kinase activity.

6. The pharmaceutical composition according to any one of claims 1 to 5, wherein the inhibitor of the expression, activity and/or function of EGFR is selected from afatinib, erlotinib, gefitinib, brigatinib, icotinib, neratinib, lapatinib, vandetanib, osimertinib, cetuximab, panitumumab, necitumumab, nimotuzumab, zalutumumab, matuzumab and combinations thereof.

7. The pharmaceutical composition according to any one of claims 1 to 6, wherein the inhibitor of the expression, activity and/or function of STAT3 is a shRNA or a PROTAC.

8. The pharmaceutical composition according to any one of claims 1 to 7, comprising a therapeutically effective amount of an inhibitor of the expression, activity and/or function of c-Raf, a therapeutically effective amount of an inhibitor of the expression, activity and/or function of EGFR, a therapeutically effective amount of an inhibitor of the expression, activity and/or function of STAT3 and optionally a pharmaceutically acceptable excipient.

9. The pharmaceutical composition according to any one of claims 1 to 8, for use in the prevention and/or treatment of cancer.

10. The pharmaceutical composition according to any one of claims 1 to 8, for use in the prevention and/or treatment of pancreatic cancer.

11. The pharmaceutical composition according to any one of claims 1 to 8, for use in the prevention and/or treatment of pancreatic intraepithelial neoplasia or of pancreatic ductal adenocarcinoma.

12. The pharmaceutical composition according to any one of claims 9 to 11, for use in tumor regression in a subject afflicted with said cancer.

13. The pharmaceutical composition according to any one of claims 9 to 11, for use in tumor regression in a subject afflicted with pancreatic ductal adenocarcinoma (PDAC).

14. The pharmaceutical composition according to any one of claims 9 to 13, for use in tumor regression in a subject who does not respond to a composition comprising only the combination of an inhibitor of the expression, activity and/or function of c-Raf and an inhibitor of the expression, activity and/or function of EGFR.
